# EUROPEAN PATENT APPLICATION

(11) **EP 2 030 522 A1**
(43) Date of publication of application: **04.03.2009**
(21) Application number: 07744850.4
(22) Date of filing: 07.06.2007
(51) Int. Cl.: A45D 20/12, A45D 1/00, A45D 20/10, A61N 1/44

(54) **CHARGED PARTICLE SUPPLYING APPARATUS**

(30) Priority: 19.06.2006 JP 2006168618
(71) Applicant: Panasonic Electric Works Co., Ltd, Kadoma-shi Osaka 571-8686 (JP)
(72) Inventor: SAIDA, Itaru c/o Panasonic Electric Works co., Ltd., Osaka 571-8686 (JP); KUMODE, Takahiro c/o Panasonic Electric Works Co., Ltd., Osaka 571-8686 (JP)
(74) Representative: Appelt, Christian W.
(86) International application number: PCT/JP2007/061520
(87) International publication number: WO 2007/148541

(57) **Abstract**

A charged particle supplying apparatus includes a discharging unit 3 that generates charged particles 1 and discharge the charged particles 1 toward a predetermined position of a human body 2 as an object, and an electric potential holding unit 4 that holds electric potential 10 of the human body 2 so that the discharged charged particles 1 are continuously induced or adsorbed by a predetermined position of the human body 2, wherein the electric potential holding unit 4 includes an insulation circuit 5 that prevents overcurrent from flowing to the object, and an electrifying unit 6 that is connected to the electric potential holding unit 4 and that is charged by the electric potential holding unit 4 is provided on a surface of a gripping portion 7 that is formed in a predetermined shape such that the gripping portion 7 can be gripped.

## Description

### TECHNICAL FIELD

The present invention relates to a charged particle supplying apparatus that generates charged particles and emits the charged particles to an object, and that holds electric potential of the object so that the charged particles can easily be induced or adsorbed by the object.

### BACKGROUND ART

A charged particle supplying apparatus that generates charged particles (negative ions or positive ions) and emits the charged particles to an object includes an electric potential holding unit. An electrifying unit connected to the electric potential holding unit is contacted with the object, the electric potential holding unit is held such that electric potential of the electrifying unit becomes opposite from electric potential of the charged particles so that the charged particles can easily be induced or adsorbed. If the charged particle supplying apparatus is used for a hair dryer or a hair brush to dry hair and emit charged particles to the hair, the hair can be moisturized or can become smooth (Patent Document 1).

In a charged particle supplying apparatus described in Patent Document 1 mentioned above, however, as insulating means between a power supply and an electrifying unit, an electronic circuit of the electric potential holding unit is only provided with a capacitor and a diode, and an insulator is only arranged between the electric potential holding unit and the electrifying unit. Therefore, when a hair dryer or a hair brush provided with the charged particle supplying apparatus falls and the electrifying unit or the insulator is cracked, there is a possibility that insulation cannot be secured sufficiently.

The present invention has been achieved to solve the problem of the background art, and an object of the present invention is to provide a safe and efficient charged particle supplying apparatus capable of always securing insulation, and capable of reliably holding electric potential of an object.
Patent Document 1: Japanese Patent Application Laid-open No. 2003-59622

### DISCLOSURE OF INVENTION

A charged particle supplying apparatus of the present invention comprises a discharging unit that generates charged particles and discharge the charged particles toward an object, and an electric potential holding unit that holds electric potential of the object such that the discharged charged particles are continuously induced or adsorbed by the object, the electric potential holding unit includes an insulation circuit that prevents overcurrent from flowing to the object, and an electrifying unit that is connected to the electric potential holding unit and that is charged by the electric potential holding unit is provided on a surface of a gripping portion that is formed in a predetermined shape such that the gripping portion can be gripped.

According to the charged particle supplying apparatus of the present invention, insulation can always and reliably be secured, a contact area between the electrifying unit and the object is secured, and electric potential of the contact object is reliably held. Therefore, it is possible to provide a safe and efficient charged particle supplying apparatus.

### BRIEF DESCRIPTION OF THE DRAWINGS

[Fig. 1] A schematic diagram showing a configuration of a charged particle supplying apparatus according to a first embodiment of the present invention.
[Fig. 2] A configuration diagram of a discharging unit of the charged particle supplying apparatus shown in Fig. 1.
[Fig. 3] A schematic diagram showing an operating state of the charged particle supplying apparatus shown in Fig. 1.
[Fig. 4] A circuit diagram (positive-electric-potential generating circuit) of an electric potential holding unit of the charged particle supplying apparatus shown in Fig. 1.
[Fig. 5] A circuit diagram (negative-electric-potential generating circuit) of an electric potential holding unit of the charged particle supplying apparatus shown in Fig. 1.
[Fig. 6] A front view of an appearance of a hair dryer having the charged particle supplying apparatus shown in Fig. 1.
[Fig. 7] An internal configuration diagram of the hair dryer having the charged particle supplying apparatus shown in Fig. 1.
[Fig. 8] A front view of an appearance of a hair brush having a charged particle supplying apparatus according to a second embodiment of the present invention.
[Fig. 9] An internal configuration diagram of the hair brush having the charged particle supplying apparatus shown in Fig. 8.
[Fig. 10] A front view of an appearance of a hair iron having a charged particle supplying apparatus according to a third embodiment of the present invention.
[Fig. 11] An internal configuration diagram of the hair iron having the charged particle supplying apparatus shown in Fig. 10.
[Fig. 12] A circuit diagram (positive-electric-potential generating circuit) of an electric potential holding unit of a charged particle supplying apparatus according to a fourth embodiment of the present invention.
[Fig. 13] A circuit diagram (negative-electric-potential generating circuit) of an electric potential holding unit of the charged particle supplying apparatus shown in Fig. 11.
[Fig. 14] A configuration diagram of an insulation circuit of the charged particle supplying apparatus shown in Fig. 12.
[Fig. 15] A configuration diagram of the insulation circuit of the charged particle supplying apparatus shown in Fig. 12.
[Fig. 16] A configuration diagram of the insulation circuit of the charged particle supplying apparatus shown in Fig. 12 (after coating insulator).
[Fig. 17] A configuration diagram of the insulation circuit of the charged particle supplying apparatus shown in Fig. 12 (after coating insulator and being bent).

### BEST MODE FOR CARRYING OUT THE INVENTION

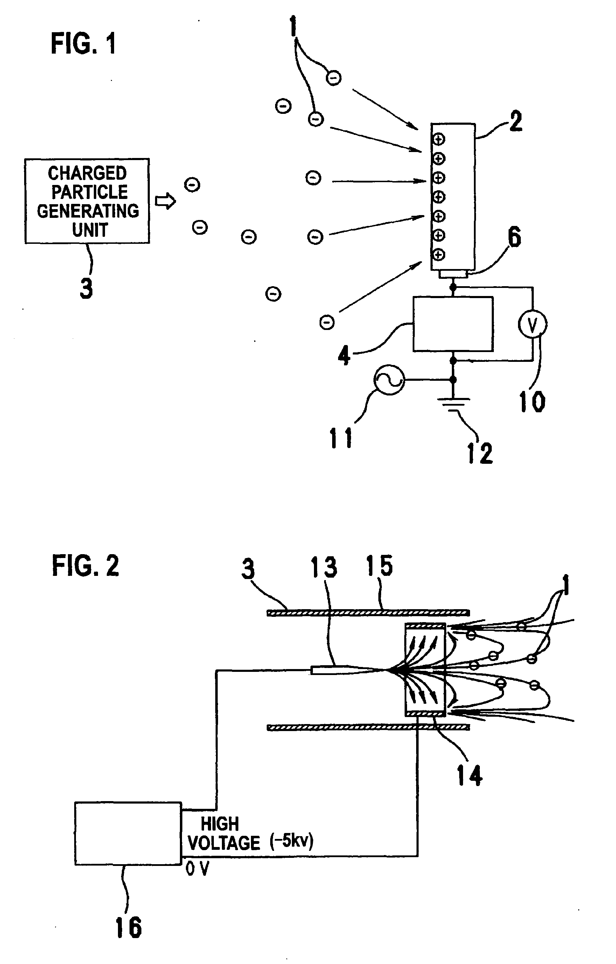

Figs. 1 to 5 show a charged particle supplying apparatus according to a first embodiment of the present invention. As shown in Figs. 1 to 5, the charged particle supplying apparatus includes a discharging unit 3 that generates charged particles 1 and emits the same toward a predetermined position of a human body 2 as an object, and an electric potential holding unit 4 that holds electric potential 10 of the human body 2 so that the emitted charged particles 1 are induced or adsorbed by the predetermined position of the human body 2 continuously. The electric potential holding unit 4 includes an insulation circuit 5 for preventing overcurrent from flowing to the object. An electrifying unit 6 that is connected to the electric potential holding unit 4 and charged by the electric potential holding unit 4 is provided on a surface of a gripping portion 7. The gripping portion 7 is formed in a predetermined shape such that the gripping portion can be gripped. The electric potential holding unit 4 and the insulation circuit 5 are provided in the gripping portion 7, and the insulation circuit 5 is provided with a transformer 8 and resistors 9.

The charged particle supplying apparatus according to the present embodiment will be explained below more specifically and in detail.

As shown in Figs. 1 to 3, the charged particle supplying apparatus includes the discharging unit 3 that generates negative air ions or positive air ions as the charged particles 1 and emits the same to the human body 2 as the object, the electric potential holding unit 4 that holds the human body 2 at the predetermined electric potential 10, and the electrifying unit 6 that is connected to the electric potential holding unit 4 and is contacted with the human body 2, and that charges the human body 2 to the predetermined electric potential 10.

The electric potential holding unit 4 that holds the electric potential 10 of the human body 2 is connected to the human body 2 by the electrifying unit 6 connected to the electric potential holding unit 4. When electric charge of the charged particles 1 is negative, the electric potential holding unit 4 provided with power supply 11 positively charges the electrifying unit 6, thereby positively charging the human body 2. A ground electric potential 12 is connected to the electric potential holding unit 4.

When electric charge of the charged particles 1 is positive, the electric potential holding unit 4 provided with the power supply 11 negatively charges the electrifying unit 6, thereby negatively charging the human body 2.

Next, the discharging unit 3 includes a needle electrode 13, a ground electrode 14, a discharge opening 15 that incorporates the needle electrode 13 and the ground electrode 14, and a high voltage generating apparatus 16. The high voltage generating apparatus 16 applies high voltage between the needle electrode 13 and the ground electrode 14 and generates charged particles 1 by corona discharge.

The needle electrode 13 is formed of a metal rod whose tip end is formed in a sharp needle shape. The ground electrode 14 is formed of metal plate, and the ground electrode 14 is arranged in front of the needle electrode 13 in the discharging unit 3.

A reference electric potential side of the high voltage generating apparatus 16 is connected to the ground electrode 14, and a high voltage side of the high voltage generating apparatus 16 is connected to the needle electrode 13. When it is desired to generate negative ions, -5 kV is output for example, and when it is desired to generate positive ions, +5 kV is output, for example.

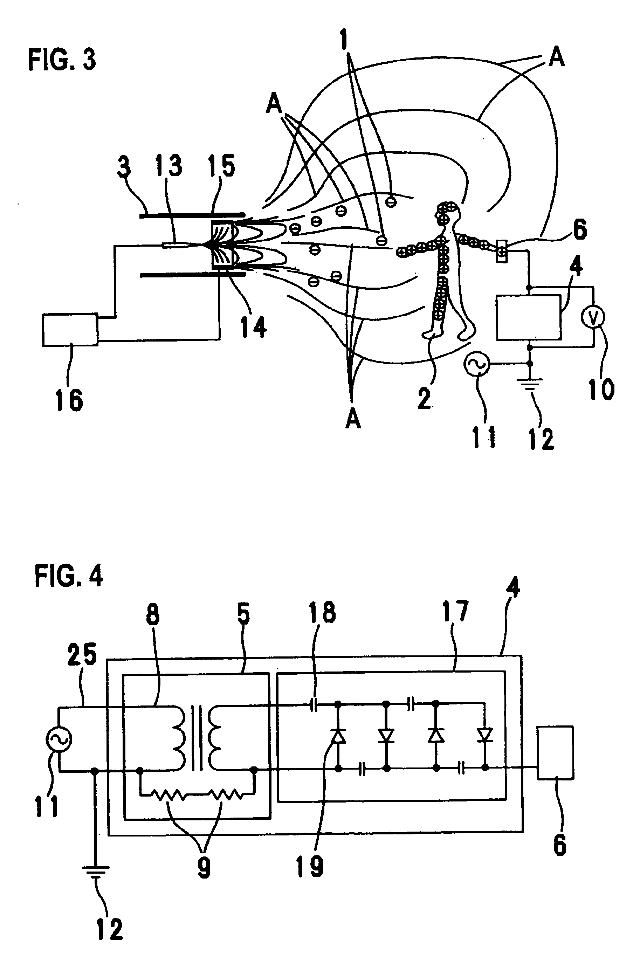

Next, as shown in Fig. 4, the insulation circuit 5 provided in the electric potential holding unit 4 is provided between the power supply 11 and a positive-electric-potential generating circuit 17 that generates positive electric potential 10. The insulation circuit 5 is provided with the transformer 8 and a plurality of the resistors 9 having high resistance values. The resistors 9 connect a primary side and a secondary side of the transformer 8, and are positioned near the ground electric potential 12.

Further, the positive-electric-potential generating circuit 17 is provided with a plurality of capacitors 18 and a plurality of diodes 19, and the positive-electric-potential generating circuit 17 generates the positive electric potential 10. The positive-electric-potential generating circuit 17 is connected to the electrifying unit 6. The positive-electric-potential generating circuit 17 charges the electrifying unit 6 with the positive electric potential 10, and also charges the human body 2 that comes into contact with the electrifying unit 6 with positive electric potential.

At this time, insulation is secured between the power supply 11, the electric potential holding unit 4 and the electrifying unit 6 connected to the electric potential holding unit 4 by the insulation circuit 5. Predetermined electric potential 10 can be given to a secondary side of the transformer 8 by the resistors 9 provided in the insulation circuit 5. When a resistance value of the resistor 9 is small, because the insulation from the ground is insufficient, it is necessary that the resistance value of the resistor 9 is high.

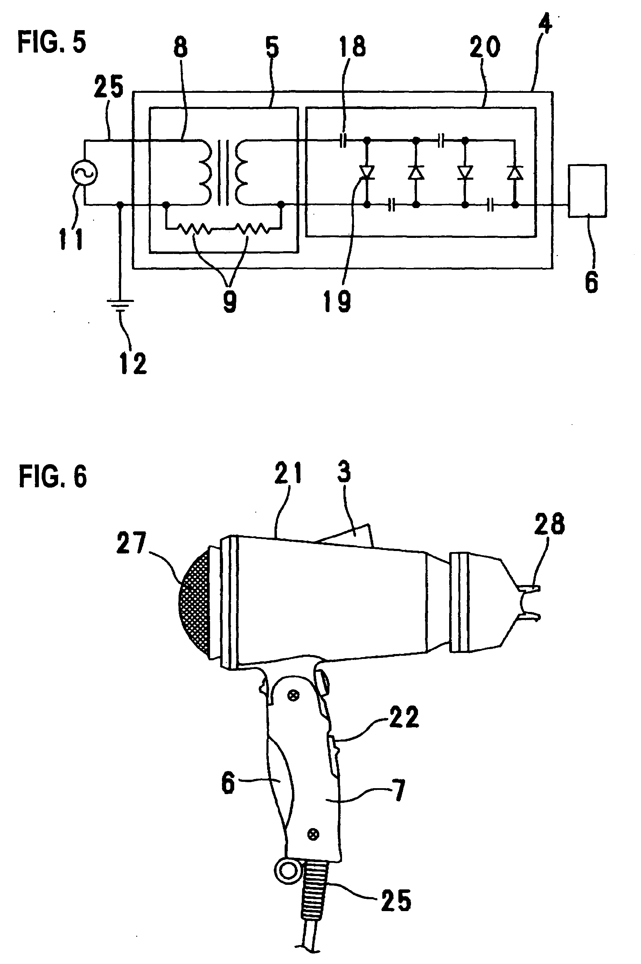

Next, in the electric potential holding unit 4, when negative electric potential 10 is held, a negative-electric-potential generating circuit 20 in which diodes are positioned in a direction opposite from the positive-electric-potential generating circuit 17 is provided as shown in Fig. 5. Therefore, the electrifying unit 6 connected to the electric potential holding unit 4 can be charged with negative electric potential, and the human body 2 that comes into contact with the electrifying unit 6 can also be charged with negative electric potential.

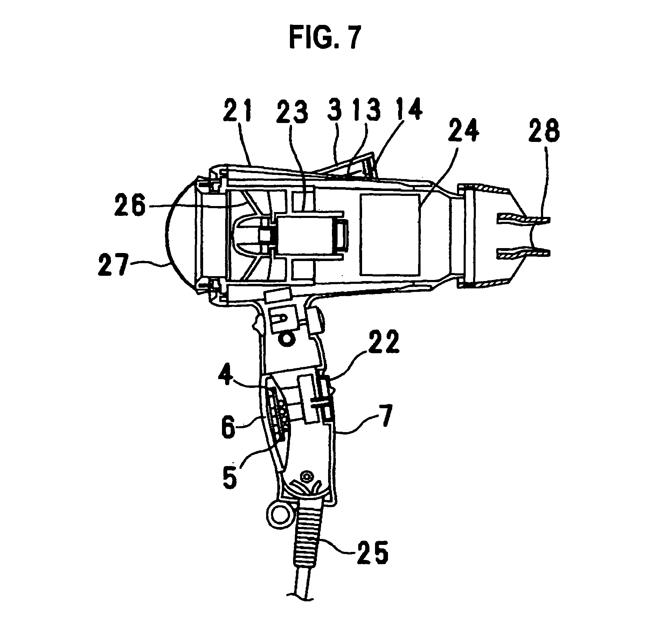

As shown in Figs. 6 and 7, when the charged particle supplying apparatus is incorporated in a dryer, the discharging unit 3 is arranged on an upper portion of a dryer body 21, and the dryer body 21 is provided at its lower side with the gripping portion 7 that is gripped by a user. The gripping portion 7 is provided at its rear side with the electrifying unit 6. The electric potential holding unit 4 having the insulation circuit 5 is provided inside of the electrifying unit 6, and this charges the electrifying unit 6 with the positive electric potential 10. A power supply cord 25 is connected to a lower side of the gripping portion 7. The electrifying unit 6 is formed in a convex curve that corresponds to the surface shape of the gripping portion 7.

An operation switch 22 is mounted on a front side of the gripping portion 7. The operation switch 22 controls driving operations and stopping operations of a motor 23 and a heater 24 provided in the dryer body 21. A fan 26 is provided behind the motor 23. If the fan 26 rotates, air sucked by a suction opening 27 is discharged from a delivery opening 28.

With respect to the above configuration, the operation of the charged particle supplying apparatus according to the first embodiment of the present invention and the dryer having the charged particle supplying apparatus will be explained by Figs. 1 to 7.

If a user grips the gripping portion 7 provided on the lower side of the dryer body 21 and turns the operation switch 22 on, the discharging unit 3 and the electric potential holding unit 4 are driven. First, the high voltage generating apparatus 16 provided in the discharging unit 3 applies -5 kV voltage to the needle electrode 13 with respect to the ground electrode 14.

If the voltage is applied, because the tip end of the needle electrode 13 is sharp, concentration of electric field occurs, corona discharge is generated near the tip end of the needle electrode 13, and charged particles 1 such as negative air ions are generated.

The discharging unit 3 is driven, the motor 23 and the heater 24 provided in the dryer body 21 are also driven, air sucked from the suction opening 27 by the fan 26 connected to the rear side of the motor 23 is heated by the heater 24 and discharged from the delivery opening 28. Charged particles 1 (negative air ions) generated and discharged in the discharging unit are supplied to the human body 2 (in this case, hair of the user) together with this discharged air, and the hair is moisturized or becomes smooth.

Meanwhile, in the electric potential holding unit 4, the positive electric potential 10 generated in the positive-electric-potential generating circuit 17 by the power supply 11 supplied from the power supply cord 25 charges the electrifying unit 6 with the positive electric potential 10. The electrifying unit 6 is connected to the electric potential holding unit 4 and provided on the rear surface of the gripping portion 7 adjacent to the electric potential holding unit 4.

At this time, because the electrifying unit 6 has the convex curved shape corresponding to the surface shape of the gripping portion 7, a user's hand and the electrifying unit 6 are closely contacted with each other, and because the contact area between the user's hand and the electrifying unit 6 can be increased, it is possible to always reliably hold the electric potential 10 of the user, i.e., the human body 2 in the positive electric potential 10.

If the human body 2 is charged with the positive electric potential 10 and held, an electric line of force A is formed toward the human body 2. Therefore, the charged particles 1 move along the electric line of force A and supplied to the human body 2. The human body 2 is charged with the positive electric potential 10 by the electric potential holding unit 4 and this state is continued. Therefore, it is possible to keep supplying the charged particles 1 to the human body 2.

The transformer 8 is provided in the insulation circuit 5. Therefore, insulation between the electric potential holding unit 4, the power supply 11 to which the power supply cord 25 is connected, and the electrifying unit 6 connected to the electric potential holding unit 4 can be secured, and predetermined electric potential 10 can be given to a secondary side of the transformer 8 by the resistors 9 provided in the insulation circuit 5.

Further, because the secondary side of the transformer 8 of the insulation circuit 5 is insulated from the power supply 11, the impedance from the ground becomes high and thus, the secondary side of the transformer 8 is susceptible to noise from outside. However, because the electric potential holding unit 4 having the insulation circuit 5 is positioned adjacent to the electrifying unit 6 in the gripping portion 7, the distance between the electric potential holding unit 4 and the electrifying unit 6 becomes short and thus, the influence of the noise from outside can be reduced to a considerably low level.

If a user turns the operation switch 22 off, the discharging unit 3 is stopped, the discharging operation of the charged particles 1 is stopped, the electric potential holding unit 4 is stopped and the holding state of the positive electric potential 10 of the human body 2 is also stopped. Because the motor 23 and the heater 24 are also stopped, the discharging operation of the heated air from the delivery opening 28 is also stopped, and the operation of the dryer is finished.

According to the first embodiment of the present invention, the electrifying unit 6 is formed in a shape such that the electrifying unit 6 is in close contact with a user's hand, and the contact area between the user's hand and the electrifying unit 6 is increased. Therefore, the positive electric potential 10 of the human body 2 can always and reliably be held.

Further, because the insulation circuit 5 is provided with the transformer 8, insulation can reliably be secured between the power supply 11 and the electrifying unit 6, and the predetermined electric potential 10 can be given to the secondary side of the transformer 8 by the resistors 9 provided in the insulation circuit 5.

Because the electric potential holding unit 4 is positioned adjacent to the electrifying unit 6 in the gripping portion 7, noise from outside can be reduced to a considerably low level.

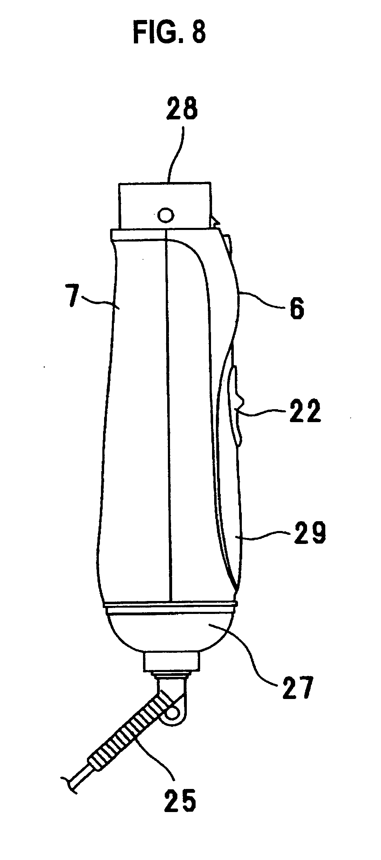

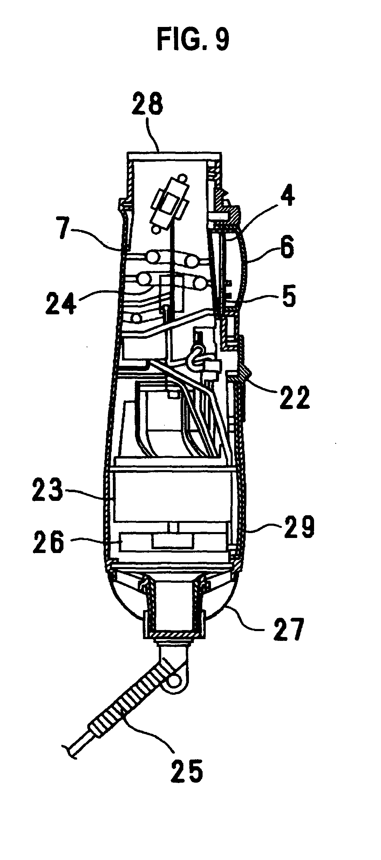

Figs. 8 and 9 show the hair brush body 29 having a charged particle supplying apparatus according to a second embodiment of the present invention. Only features of the embodiment different from the first embodiment will be explained, and for the same configurations and effects as those of the first embodiment, the explanations of the first embodiment will be incorporated herein.

As shown in Figs. 8 and 9, the second embodiment of the present invention is different from the first embodiment in that the gripping portion 7 is formed on an upper portion of the hair brush body 29, the electrifying unit 6 is positioned on the gripping portion 7 and on the upper side of the operation switch 22. The power supply cord 25 is connected near the suction opening 27.

Based on these differences, the operations of the charged particle supplying apparatus and the hair brush having the charged particle supplying apparatus according to the second embodiment of the present invention will be explained with reference to Figs. 1 to 5, 8 and 9.

If a user grips the gripping portion 7 provided on the upper portion of the hair brush body 29 and turns the operation switch 22 on, the discharging unit 3 (not shown in Figs. 8 and 9) and the electric potential holding unit 4 are driven, and -5 kV voltage, for example, is first applied from the high voltage generating apparatus 16 provided in the discharging unit 3 to the needle electrode 13 with respect to the ground electrode 14.

If the voltage is applied, because the tip end of the needle electrode 13 is sharp, concentration of electric field occurs, corona discharge is generated near the tip end of the needle electrode 13, and the charged particles 1 such as negative air ions are generated.

The discharging unit 3 is driven, the motor 23 and the heater 24 provided in the hair brush body 29 are also driven, air sucked from the suction opening 27 by the fan 26 connected to the rear side of the motor 23 is heated by the heater 24 and discharged from the delivery opening 28. The charged particles 1 (negative air ions) generated and discharged in the discharging unit 3 are supplied to the human body 2 (in this case, hair of the user) together with this discharged air, and the hair is moisturized or becomes smooth.

Meanwhile, in the electric potential holding unit 4, the positive electric potential 10 generated in the positive-electric-potential generating circuit 17 by the power supply 11 supplied from the power supply cord 25 charges the electrifying unit 6 with the positive electric potential 10. The electrifying unit 6 is connected to the electric potential holding unit 4 and provided on the rear surface of the gripping portion 7 adjacent to the electric potential holding unit 4.

At this time, because the electrifying unit 6 has the convex curved shape corresponding to the surface shape of the gripping portion 7, a user's hand and the electrifying unit 6 are closely contacted with each other, and because the contact area between the user's hand and the electrifying unit 6 can be increased, it is possible to always reliably hold the electric potential 10 of the user, i.e., the human body 2 in positive electric potential.

If the human body 2 is charged with the positive electric potential 10 and held, the electric line of force A is formed toward the human body 2. Therefore, the charged particles 1 move along the electric line of force A and supplied to the human body 2. The human body 2 is charged with the positive electric potential 10 by the electric potential holding unit 4 and this state is continued. Therefore, it is possible to keep supplying the charged particles 1 to the human body 2.

The transformer 8 is provided in the insulation circuit 5. Therefore, insulation between the electric potential holding unit 4, the power supply 11 to which the power supply cord 25 is connected, and the electrifying unit 6 connected to the electric potential holding unit 4 can be secured, and the predetermined electric potential 10 can be given to a secondary side of the transformer 8 by the resistors 9 provided in the insulation circuit 5.

Further, because the secondary side of the transformer 8 of the insulation circuit 5 is insulated from the power supply 11, the impedance from the ground becomes high and thus, the secondary side of the transformer 8 is susceptible to noise from outside. However, because the electric potential holding unit 4 having the insulation circuit 5 is positioned adjacent to the electrifying unit 6 in the gripping portion 7, the distance between the electric potential holding unit 4 and the electrifying unit 6 becomes short and thus, the influence of the noise from outside can be reduced to a considerably low level.

If a user turns the operation switch 22 off, the discharging unit 3 is stopped, the discharging operation of the charged particles 1 is stopped, the electric potential holding unit 4 is stopped and the holding state of the positive electric potential 10 of the human body 2 is also stopped. Because the motor 23 and the heater 24 are also stopped, the discharging operation of the heated air from the delivery opening 28 is also stopped, and the operation of the hair brush is finished.

As described above, according to the second embodiment of the present invention, the electrifying unit 6 is formed in a shape such that the electrifying unit 6 is in close contact with a user's hand, and the contact area between the user's hand and the electrifying unit 6 is increased. Thus, the positive electric potential 10 of the human body 2 can always and reliably be held.

Further, because the insulation circuit 5 is provided with the transformer 8, insulation can reliably be secured between the power supply 11 and the electrifying unit 6, and the predetermined electric potential 10 can be given to the secondary side of the transformer 8 by the resistors 9 provided in the insulation circuit 5.

Because the electric potential holding unit 4 is positioned adjacent to the electrifying unit 6 in the gripping portion 7, noise from outside can be reduced to a considerably low level.

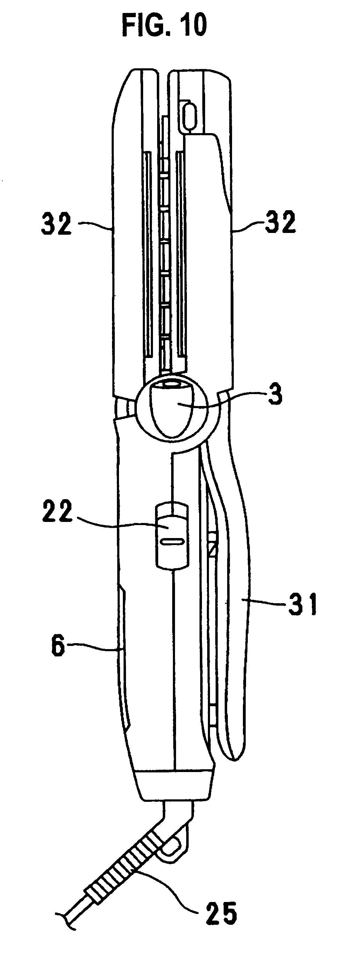

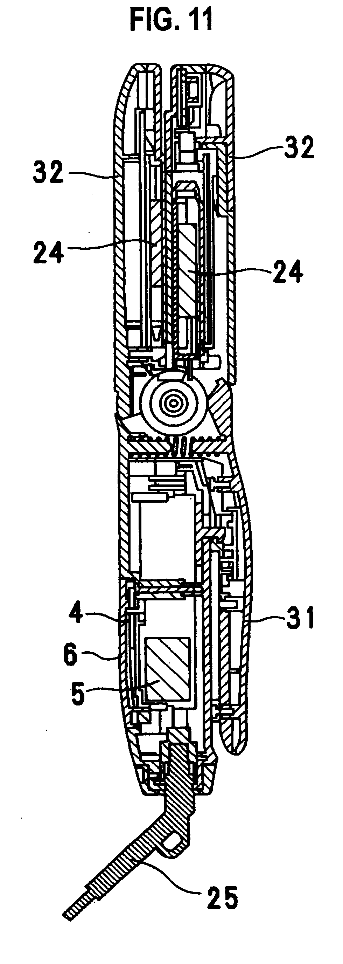

Figs. 10 and 11 show the hair brush body 29 having a charged particle supplying apparatus according to a third embodiment of the present invention. Only features of the embodiment different from the first embodiment will be explained, and for the same configurations and effects as those of the first embodiment, the explanations of the first embodiment will be incorporated herein.

As shown in Figs. 10 and 11, the third embodiment of the present invention is different from the first embodiment in that the gripping portion 7 is provided on a lower portion of a hair iron body 30. The electrifying unit 6 is positioned below the operation switch 22 in the gripping portion 7.

Unlike the dryer, the hair brush body 29 does not have the motor 23 and the fan 26, but has ironing units 32 that can be opened and closed by a lever 31. The heater 24 is arranged in the ironing unit 32, and heats user's hair nipped by the ironing units 32. The discharging units 3 that discharge the charged particles 1 are provided on both sides of the hair iron body 30.

Based on these differences, the operations of the charged particle supplying apparatus and the hair iron having the charged particle supplying apparatus according to the third embodiment of the present invention will be explained with reference to Figs. 1 to 5, 10 and 11.

In a state that the user grips the gripping portion 7 provided on the lower portion of the hair iron body 30, the user operates the lever 31 to open the ironing units 32 and nip hair between the ironing units 32 and the ironing units 32 are closed, if a user turns the operation switch 22 on, the discharging units 3 provided on both sides of the hair iron body 30 and the electric potential holding unit 4 arranged in the electrifying unit 6 are driven, and -5 kV voltage, for example, is applied from the high voltage generating apparatus 16 provided in the discharging unit 3 to the needle electrode 13 with respect to the ground electrode 14.

If the voltage is applied, because the tip end of the needle electrode 13 is sharp, concentration of electric field occurs, corona discharge is generated near the tip end of the needle electrode 13, and the charged particles 1 such as negative air ions are generated.

The discharging units 3 are driven, the heaters 24 provided in the ironing units 32 are driven, and hair nipped by the ironing units 32 is heated and dressed. At this time, the charged particles 1 (negative air ions) generated and discharged in the discharging units 3 are supplied to the human body 2 (in this case, hair of the user) together with this discharged air, and the hair is moisturized or becomes smooth.

Meanwhile, in the electric potential holding unit 4, the positive electric potential 10 generated in the positive-electric-potential generating circuit 17 by the power supply 11 supplied from the power supply cord 25 charges the electrifying unit 6 with the positive electric potential 10. The electrifying unit 6 is connected to the electric potential holding unit 4 and provided on the rear surface of the gripping portion 7 adjacent to the electric potential holding unit 4.

At this time, because the electrifying unit 6 has the convex curved shape corresponding to the surface shape of the gripping portion 7, a user's hand and the electrifying unit 6 are closely contacted with each other, and because the contact area between the user's hand and the electrifying unit 6 can be increased, it is possible to always reliably hold the electric potential 10 of the user, i.e., the human body 2 in positive electric potential.

If the human body 2 is charged with the positive electric potential 10 and held, the electric line of force A is formed toward the human body 2. Therefore, the charged particles 1 move along the electric line of force A and supplied to the human body 2. The human body 2 is charged with the positive electric potential 10 by the electric potential holding unit 4 and this state is continued. Therefore, it is possible to keep supplying the charged particles 1 to the human body 2.

The transformer 8 is provided in the insulation circuit 5. Therefore, insulation between the electric potential holding unit 4, the power supply 11 to which the power supply cord 25 is connected, and the electrifying unit 6 connected to the electric potential holding unit 4 can be secured, and the predetermined electric potential 10 can be given to a secondary side of the transformer 8 by the resistors 9 provided in the insulation circuit 5.

Further, because the secondary side of the transformer 8 of the insulation circuit 5 is insulated from the power supply 11, the impedance from the ground becomes high and thus, the secondary side of the transformer 8 is susceptible to noise from outside. However, because the electric potential holding unit 4 having the insulation circuit 5 is positioned adjacent to the electrifying unit 6 in the gripping portion 7, the distance between the electric potential holding unit 4 and the electrifying unit 6 becomes short and thus, the influence of the noise from outside can be reduced to a considerably low level.

If the user turns the operation switch 22 off, the discharging units 3 are stopped, the discharging operation of the charged particles 1 is stopped, the electric potential holding unit 4 is stopped, and the holding operation of the positive electric potential 10 of the human body 2 is also stopped. Because the heaters 24 are also stopped, the heating operation of hair nipped by the ironing units 32 is stopped, and the operation of the hair iron is finished.

As described above, according to the third embodiment of the present invention, the electrifying unit 6 is formed in a shape such that the electrifying unit 6 is in close contact with a user's hand, and the contact area between the user's hand and the electrifying unit 6 is increased. Therefore, the positive electric potential 10 of the human body 2 can always and reliably be held.

Because the insulation circuit 5 is provided with the transformer 8, insulation can reliably be secured between the power supply 11 and the electrifying unit 6, and the predetermined electric potential 10 can be given to the secondary side of the transformer 8 by the resistors 9 provided in the insulation circuit 5.

Further, because the electric potential holding unit 4 is positioned adjacent to the electrifying unit 6 in the gripping portion 7, noise from outside can be reduced to a considerably low level.

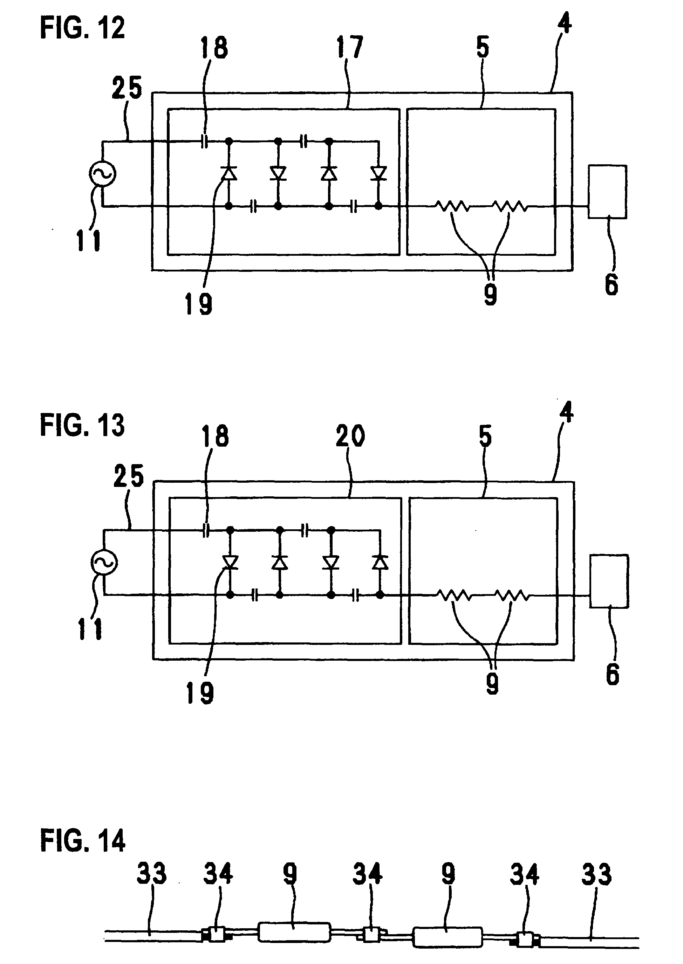

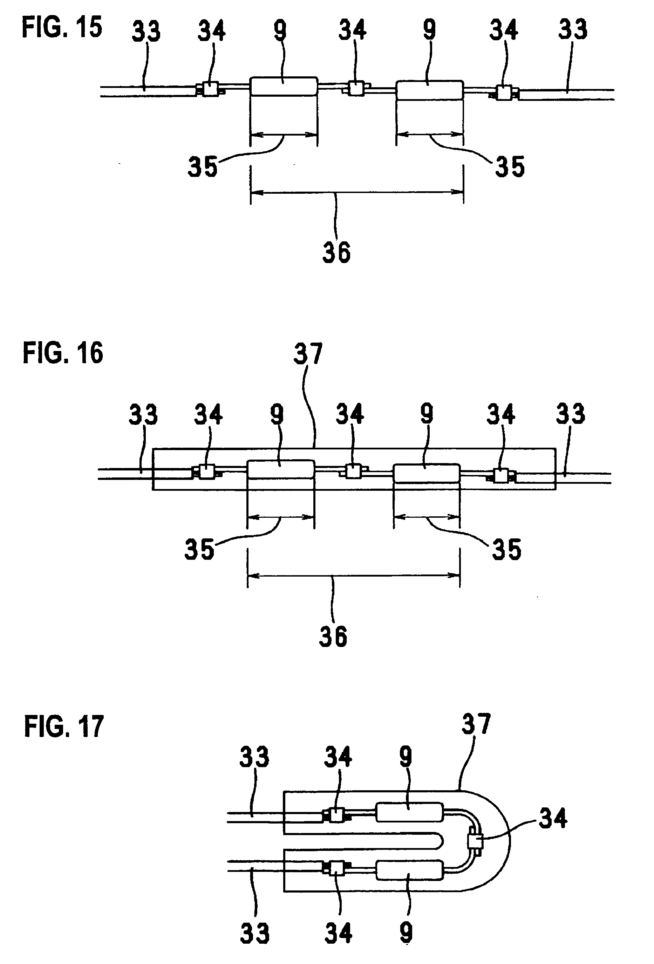

Figs. 12 to 17 show the electric potential holding unit 4 of a charged particle supplying apparatus according to a fourth embodiment of the present invention. Only features of the embodiment different from the first embodiment will be explained, and for the same configurations and effects as those of the first embodiment, the explanations of the first embodiment will be incorporated herein.

As shown in Figs. 12 to 17, the fourth embodiment of the present invention is different from the first embodiment in that the insulation circuit 5 is arranged between the electrifying unit 6 and the positive-electric-potential generating circuit 17 or the negative-electric-potential generating circuit 20, and that the insulation circuit 5 includes a plurality of the resistors 9.

As shown in Figs. 14 to 17, the resistors 9 are connected to each other through caulking irons 34, and the resistors 9 and lead wires 33 are connected to each other through the caulking irons 34. Base insulations 35 are secured by the resistors 9, and because the resistors 9 are provided, a double insulation 36 is secured.

As described above, because insulation can be secured using the resistors 9, the insulation circuit 5 can be remarkably downsized, and the insulation circuit 5 can be arranged in a small space in the gripping portion 7.

Further, ends of the lead wires 33 and the resistors 9 including the caulking irons 34 are coated with insulator 37 of vinyl chloride or the like. A creepage distance for insulation can be secured even if the resistors 9 are bent. Therefore, the insulation circuit 5 can be arranged in an even smaller space with an inexpensive configuration while sufficiently securing insulation.

In the embodiments of the present invention, the charged particles 1 having the negative electric potential 10 are generated in the discharging unit 3, and the positive electric potential 10 are held in the electric potential holding unit 4, but the charged particles 1 having the positive electric potential 10 can be generated in the discharging unit 3, and the negative electric potential 10 can be held in the electric potential holding unit 4.

In this case, as shown in Figs. 5 and 13, the electric potential holding unit 4 is provided with the negative-electric-potential generating circuit 20, and in the discharging unit 3, +5 kV voltage can be applied to the needle electrode 13 with respect to the ground electrode 14.

Both the positive-electric-potential generating circuit 17 and negative-electric-potential generating circuit 20 can be provided in the electric potential holding unit 4, and the electric potential 10 of the electric potential holding unit 4 can be switched according to the electric potential 10 of the generated charged particles 1.

### INDUSTRIAL APPLICABILITY

The present invention can be applied to a hair dryer and a hair brush.

## Claims

1. A charged particle supplying apparatus comprising a discharging unit that generates charged particles and discharge the charged particles toward an object, and an electric potential holding unit that holds electric potential of the object such that the discharged charged particles are continuously induced or adsorbed by the object, wherein the electric potential holding unit includes an insulation circuit that prevents overcurrent from flowing to the object, and an electrifying unit that is connected to the electric potential holding unit and that is charged by the electric potential holding unit is provided on a surface of a gripping portion that is formed in a predetermined shape such that the gripping portion can be gripped.

2. The charged particle supplying apparatus according to claim 1, wherein the electric potential holding unit and the insulation circuit are provided in the gripping portion.

3. The charged particle supplying apparatus according to claim 1 or 2, wherein the insulation circuit is provided with a transformer and a resistor.

4. The charged particle supplying apparatus according to claim 1 or 2, wherein the insulation circuit is provided with a plurality of resistors.

5. The charged particle supplying apparatus according to claim 4, wherein insulators are arranged on surfaces of the plurality of resistors.
